Europäisches Patentamt

European Patent Office

Office européen des brevets

(19)

(11) Veröffentlichungsnummer: **0 288 698**

**A2**

(12) # EUROPÄISCHE PATENTANMELDUNG

(21) Anmeldenummer: 88103713.9

(22) Anmeldetag: 09.03.88

(51) Int. Cl.⁴: **A61B 19/00 , A61B 6/04**

(30) Priorität: 30.04.87 DE 3714397

(43) Veröffentlichungstag der Anmeldung:
02.11.88 Patentblatt 88/44

(84) Benannte Vertragsstaaten:
**CH DE FR GB IT LI NL SE**

(71) Anmelder: **DORNIER MEDIZINTECHNIK GMBH**
**Postfach 1128**
**D-8034 Germering 1(DE)**

(72) Erfinder: **Wess, Othmar, Dr. Dipl.-Phys.**
**Max-Nadler-Strasse 17**
**D-8000 München 81(DE)**
Erfinder: **Denk, Roland, Dipl.-Phys.**
**Rankestrasse 11**
**D-8000 München 40(DE)**
Erfinder: **Restle, Karl-Heinz, Ing-grad.**
**Herrgottsweiler 3**
**D-7992 Tettnang(DE)**
Erfinder: **Weiler, Herbert**
**Bniesstrasse 11a**
**D-8031 Alling(DE)**

(74) Vertreter: **Landsmann, Ralf, Dipl.-Ing.**
**DORNIER GMBH - Patentabteilung - Kleeweg**
**3**
**D-7990 Friedrichshafen 1(DE)**

(54) Universeller medizinischer Arbeitsplatz.

(57) Medizinischer Arbeitsplatz, wobei alle zur Ultraschall-und Röntgendiagnostik und/oder zur Stosswellenbehandlung von Nieren-und Gallenkonkrementen oder zu Standard-Untersuchungen, insbesondere der Urologie, erforderlichen Geräte vorgesehen und so um einen im räumlichen Zentrum stehenden Zentralsockel (2) herum angeordnet und von dort aus erreichbar sind, dass die Lage eines Patienten (30) auf einer Patientenliege (34) des Arbeitsplatzes konstant bleibt.

Fig. 6

## Universeller medizinischer Arbeitsplatz

Die Erfindung betrifft einen medizinischen Arbeitsplatz für die Stosswellentherapie von Nieren- und Gallenkonkrementen sowie zur Ultraschall-und Röntgendiagnostik und für allgemeine Untersuchungen, insbesondere urologische Untersuchungen.

Im Vorfeld und bei der Nachbehandlung der Nieren und Gallenlithotripsie durch Stosswellen müssen verschiedene Manipulationen am Patienten durchgeführt werden. Auch auch bei allgemeinen urologischen Untersuchungen und Behandlungen muß der Patient in unterschiedliche Stellungen gebracht werden. In beiden Fällen ist häufig die Anwendung von Röntgen-und Ultraschallgeräten erforderlich. Um die untersuchungs-oder behandlungsrelevanten Körperteile aus allen Richtungen behandeln zu können, ist häufig eine Umlagerung des Patienten notwendig, beispielsweise wenn der Zertrümmerungserfolg einer Nierensteinbehandlung durch eine hochqualitative Röntgenaufnahme ausserhalb des Lithotripters ermittelt werden soll.

Eine spezielle Problematik besteht dabei darin, dass Röntgeneinrichtung und Stosswelleneinrichtung räumlich so weit voneinander getrennt sind, dass der Patient zwischen beiden Einrichtungen umgelagert werden muß.

Der Erfindung liegt die Aufgabe zugrunde, einen Arbeitsplatz zu schaffen, der es ermöglicht, eine Röntgen-und/oder Ultraschallpositioniereinrichtung, eine Stosswelleneinrichtung und/oder einen urologischen Behandlungsplatz räumlich so zu verbinden, dass sich die Einrichtungen gegenseitig in ihrer Bewegungsfreiheit nicht einschränken, und zwischen den Einrichtungen der Patient nicht umgelagert werden muß.

Die Aufgabe wird durch den in Haupt-und Unteransprüchen charakterisierten Arbeitsplatz gelöst.

Der erfindungsgemässe Arbeitsplatz umfasst eine Geräteanordnung, bei der Ortungs-/Diagnoseeinrichtung und Therapieeinrichtung räumlich voneinander getrennt sind. Beide Einrichtungen liegen aber dennoch räumlich so zueinander, dass der Transport des Patienten zu den einzelnen Einrichtungen lediglich in einem Schwenken der Patientenliege besteht, wobei die einzelnen im Schwenkbereich liegenden Positionen der Liege einer exakt definierten Zuordnung unterliegen, und der Patient so auf der Liege seine einmal eingenommene Position nicht mehr verändert. Dies ist insbesondere dann wichtig, wenn eine Narkose des Patienten vorliegt.

Die modular aufgebaute Anordnung besteht im wesentlichen aus vier Teilen:
- einem zentral angeordneten Sockel als Träger der Patientenliege,
- einer am Sockel montierten Ultraschall-Ortungseinrichtung,
- einer Stosswellenerzeugungseinrichtung mit Koppelkissen und
- einer Röntgenlage.

Arzt und medizinisches Personal haben bei vorhandenem Konzept jederzeit freien Zugang zum Patienten. Die Anästhesie bekommt eine feste Position im Behandlungsraum, da bei allen Schwenkungen der Patientenliege der Kopfteil ortsfest bleibt. Bei der Behandlung von Konkrementen und der dabei erforderlichen Ortung dieser Konkremente im menschlichen Körper können verschiedene Ortungssysteme Verwendung finden. So kann sowohl mit Ultraschall als auch mit Röntgenstrahlen die Position des zu zertrümmernden Konkrements erkannt werden. Dabei ist mit der Röntgenanlage eine freie Wahl der Röntgenortungsebenen gegeben, bei der Verwendung einer Untertisch-und Obertischanlage ist sogar die Nutzung der Röntgenröhre variabel.

Der erfindungsgemässe medizinische Arbeitsplatz ist insbesondere für folgende Anwendungen gedacht:
- Nierensteinzertrümmerung
- Gallensteinzertrümmerung
- allgemeine Ultraschalldiagnostik
- allgemeine Röntgendiagnostik, insbesondere alle wesentlichen Standard-Untersuchungen der Urologie
- Kathetriesierung unter Röntgenkontrolle
- Röntgen-Kassettenaufnahme in der Urologie und Oberbauchdiagnostik
- Röntgendurchleuchtung
- Perkutane Litholapaxie links-oder rechtsseitig mit wahlweise Untertisch-bzw. Obertisch-Röntgenröhre
- Perkutane Litholapaxie unter Ultraschallkontrolle
- Ultraschall bzw. röntgengeführte Punktion.

Die Erfindung wird anhand von Figuren näher erläutert.

Es zeigen:

Figur 1 eine Seitenansicht des Arbeitsplatzes mit Ultraschall-Ortungseinrichtung und Stosswellenerzeuger,

Figur 2 eine Draufsicht nach Figur 1 mit zusätzlichem Steuerpult,

Figur 3 eine Seitenansicht des Arbeitsplatzes bei der Verwendung einer Röntgenanlage,

Figur 4 eine Draufsicht nach Figur 3 mit zusätzlichem Steuerpult,

Figur 5 eine Seitenansicht des Arbeitsplatzes bei der Verwendung der Röntgenanlage und veränderter Liegenposition,

Figur 6 eine Draufsicht nach Figur 5 mit zusätzlichem Steuerpult und Darstellung der Verwendung der Patientenliege bei einer urologischen Untersuchung.

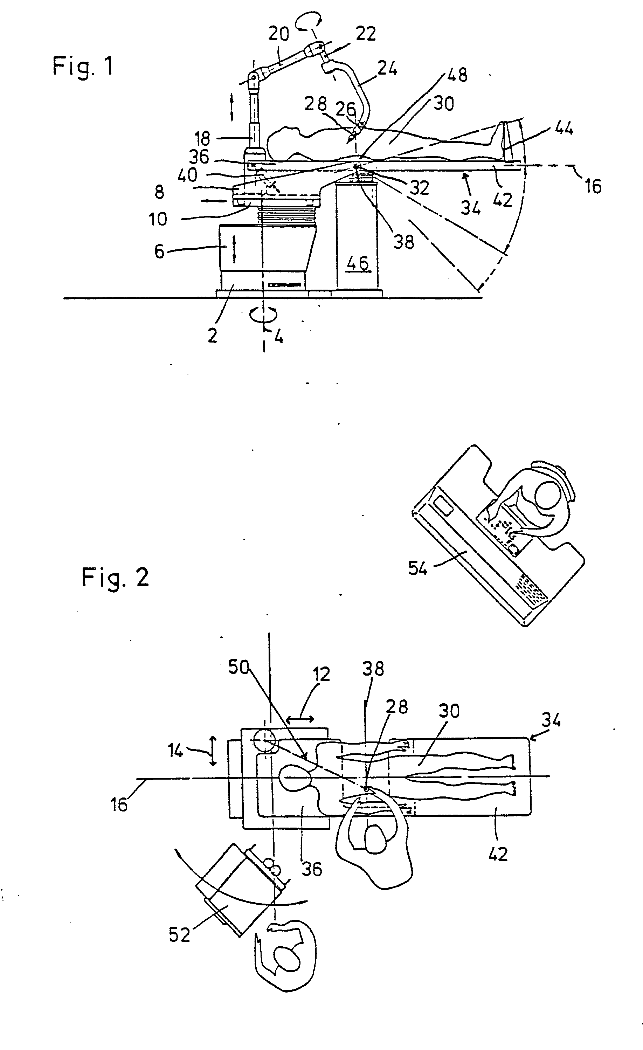

Figur 1 zeigt eine Seitenansicht der Anordnung mit sich im räumlichen Zentrum der Anordnung befindlichen Sockel 2. Auf diesem Sockel 2 ist ein um seine z-Achse 4 herum drehbares Bauteil 6 gelagert, das einen Winkel von vorzugsweise 270° überstreicht. Gleichzeitig ist das Bauteil 6 auch entlang der z-Achse 4 vertikal verstellbar. Auf dem Bauteil 6 ist ein Tragarm 8 mit Bodenplatte 10 montiert. Dieser Tragarm 8 lässt sich horizontal in x-Richtung 12 und y-Richtung 14 verschieben, gleichzeitig ist eine Kippung um die Längsachse 16 möglich (siehe auch Figur 2). Oben auf der Bodenplatte 8 ist ein längenvariabler Tragarm 18 befestigt. Die Längenveränderung kann beispielsweise über teleskopartig ausziehbare Glieder geschehen. An den Tragarm 18 sind vier weitere Armglieder 20 bis 26 nacheinanderliegend angeschlossen, wobei der letzte Arm 26 einen Ultraschallkopf 28 trägt. Durch die Anordnung der Arme 18 bis 26 bedingt, ist die Bewegungsfreiheit des Ultraschallkopfes 28 so gestaltet, dass damit jede Stelle der zur Behandlung anstehenden Körperteile eines Patienten 30 erreicht werden kann.

Im Tragarm 8 befindet sich ein Gelenk 32, an der eine Patientenliege 34 befestigt ist. Die Patientenliege 34 besteht in der gezeigten Version aus drei Teilen. Der erste Teil 36 ist über eine Achse 38 direkt mit dem Tragarm 8 verbunden. Um diese Achse 38 ist die gesamte Liege 34 kippbar, so dass Kopf-und Fußteil des Patienten 30 sowohl auf gleicher, als auch auf unterschiedlicher Höhe liegen können. Dabei liegt der Patient 30 aber immer gestreckt in einer Ebene. Die Neigung der Patientenliege 34 wird über einen Stellmotor 40 verändert. Auf dem Teil 36 kommen der Kopf, die Schulterpartie, die Arme und das Becken des Patienten 30 zu liegen, während auf dem am ersten Teil 36 befestigten zweiten Teil 42 die Beine ruhen. Die Füße können dabei an einem dritten Teil 44 abgestützt werden. Die Befestigung des zweiten Teils 42 am ersten Teil 36 kann beispielsweise durch Einstecken des zweiten Teils 42 in den ersten Teil 36 geschehen.

Neben dem Sockel 2 ist eine Stosswellenerzeugungseinrichtung 46 vorgesehen, die beispielsweise als eine runde Säule mit am oberen Ende angebrachtem Koppelkissen 48 ausgestaltet ist. Dieses Koppelkissen 48 passt in eine Aussparung im ersten Teil 36 der Patientenliege 34, so dass das Koppelkissen 48 unmittelbar an der Körper des Patienten 30 angekoppelt werden kann.

Figur 2 zeigt die Konstellation des Arbeitsplatzes nach Figur 1 in einer Draufsicht. Hierbei ist die Lage des Patienten 30 auf den beiden Teilen 36 und 42 der Patientenliege 34 gut zu erkennen. Die skizzierten Armglieder 20 bis 26 und der Ultraschallkopf 28 der Ultraschallanlage 50 werden über die zu untersuchenden Körperteile 30 geführt. Das mit dem Ultraschallkopf 28 aufgenommene Bild wird auf einem ebenfalls verschwenkbaren Monitor 52 sichtbar gemacht. Die Steuerung und Überwachung der gesamten Anlage wird von einem Steuerpult 54 aus vorgenommen, wobei auch daran gedacht ist, Positionierung, Steuerung und Überwachung elektronisch unterstützt und an dem Steuerpult 54 anzeigbar auszuführen.

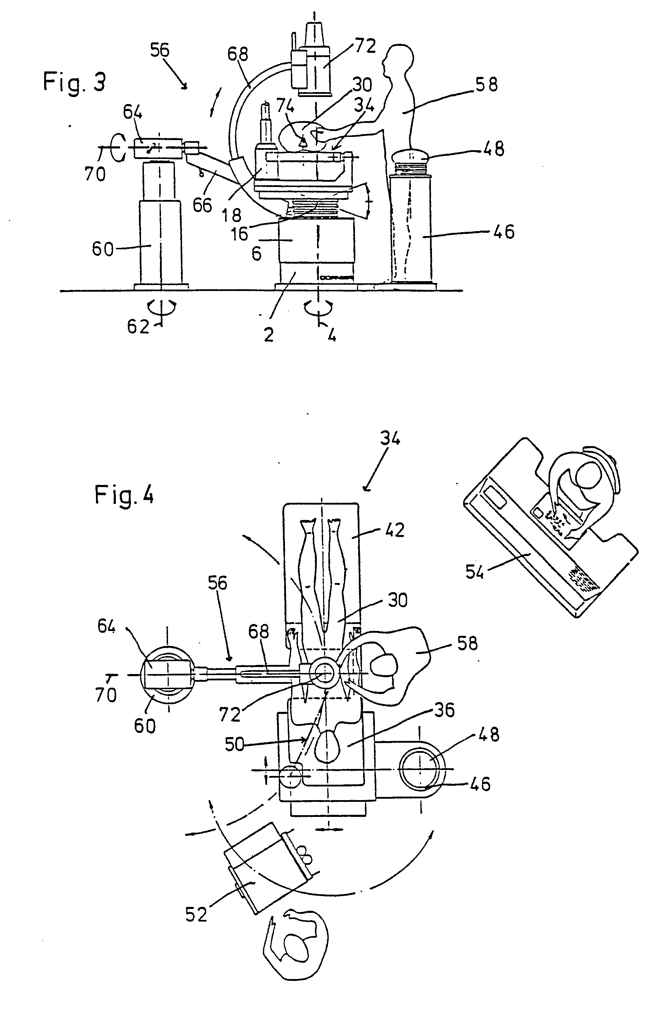

Figur 3 zeigt den Arbeitsplatz beim Einsatz einer Röntgenanlage 56, die in den Figuren 1 und 2 nicht explizit gezeigt ist. Dazu wird die Patientenliege 34 beispielsweise um 90° aus ihrer Stosswellenbehandlungsposition herausgeschwenkt. Die Säule der Stosswellenerzeugungseinrichtung 46 mit Koppelkissen 48 ist rechts vor der behandelnden Person 58 zu sehen. Links vom Bauteil 6 mit Patientenliege 34 ist eine Standsäule 60 mit einem um wenigstens 180° um eine z-Achse 62 drehbaren Oberteil 64 der Röntgenanlage 56 aufgestellt, wobei an dem Oberteil 64 die Halterung 66 des bogenförmigen Armes 68 angebracht ist, wobei die Halterung 66 um eine senkrecht zur Achse 62 stehende Achse 70 schwenkbar ist.

Das Röntgenaufnahmegerät 72 lässt sich auf dem bogenförmigen Arm 68 so um die Längsachse 16 herum bewegen, dass alle Seiten des Körpers des Patienten 30 durchstrahlt werden können. Ebenfalls bruchstückhaft dargestellt ist der Tragarm 18 der Ultraschallanlage 50. Deutlich zu sehen ist auch die Möglichkeit, die Patientenliege 34 um Längsachse 16 zu kippen, damit die Wirbelsäule 74 des Patienten 30 oder andere Hindernisse bei der Stosswellenbehandlung nicht die Konkremente verdecken.

Figur 4 zeigt den Arbeitsplatz nach Figur 3 in der Draufsicht. Schwenkbereich der Röntgenanlage 56, Monitor 52 und Steuerpult 54 sind gut zu erkennen.

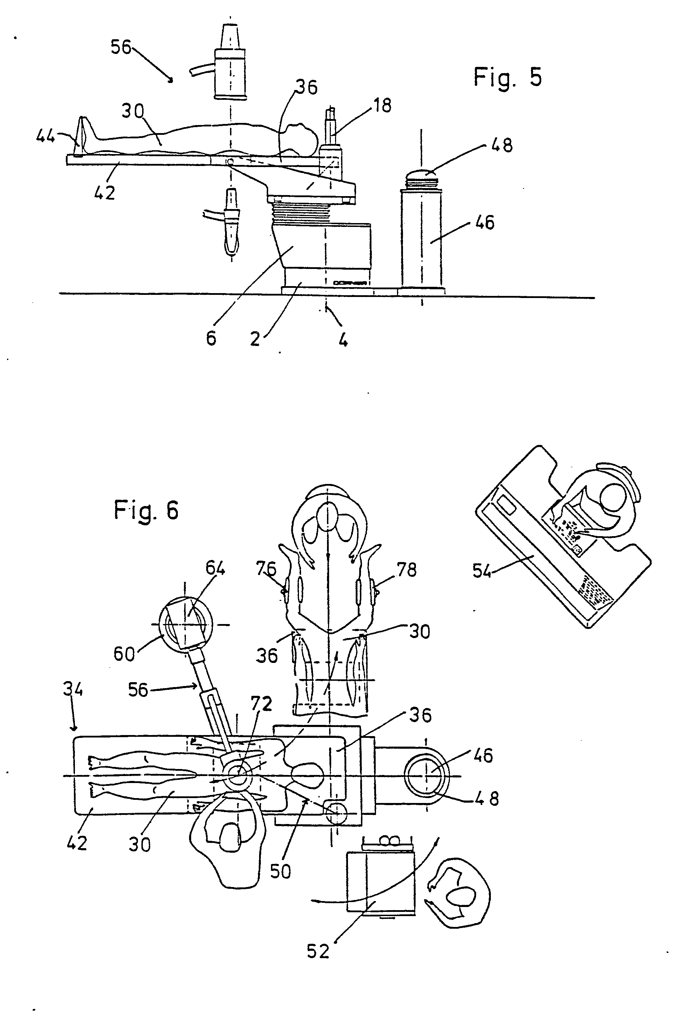

Figur 5 zeigt den Arbeitsplatz aus der Seitenansicht mit veränderter Patientenliegenposition und ausschnittsweiser Darstellung der Röntgenanlage 56. Die Verstellmöglichkeiten der Anlage in Figur 3 bleiben unbenommen.

Figur 6 zeigt den Arbeitsplatz nach Figur 5 in der Draufsicht. Hierbei ist zusätzlich die Möglichkeit aufgezeigt, den zweiten Teil 42 der Patientenliege 34 durch zwei Beinstützen 76 und 78 zu ersetzen, die ebenfalls in den ersten Teil 36 der Patientenliege 34 einsteckbar sind. Dadurch ist es einer behandelnden Person auch möglich, urologische Untersuchungen und -behandlungen durchzuführen. In Figur 6 ist die Patientenliege 34 um 180° aus der Stosswellenbehandlungsposition herausgeschwenkt, wobei auch ein Schwenken um 90° in die andere Richtung den Patienten 30 in den Bereich der Röntgenanlage 56 bringt.

Zur Aufzeichnung der Röntgen-und Ultraschallbilder kann eine entsprechende Aufnahmevorrichtung vorgesehen sein, beispielsweise ein Kassettenaufnahmegerät.

## Ansprüche

1. Medizinischer Arbeitsplatz, **dadurch gekennzeichnet,** dass alle zur Ultraschall-und Röntgendiagnostik und/oder zur Stosswellenbehandlung von Nieren und Gallenkonkrementen oder zu Standard-Untersuchungen, insbesondere der Urologie, erforderlichen Geräte vorgesehen und so um einen im räumlichen Zentrum stehenden Zentralsockel (2) herum angeordnet und von dort aus erreichbar sind, dass die Lage eines Patienten (30) auf einer Patientenliege (34) des Arbeitsplatzes konstant bleibt.

2. Medizinischer Arbeitsplatz nach Anspruch 1, dadurch gekennzeichnet, dass ein in einem räumlichen Zentrum stehender, drehbarer und vertikal verstellbarer Sockel (2) mit darauf gelagertem Bauteil (6) vorgesehen ist, an dem ein horizontal in x- und y-Richtung (12, 14) beweglicher und um die Längsachse (16) kippbarer Tragarm (8) mit Bodenplatte (10) zur Befestigung der Patientenliege (34) und ein weiterer Tragarm (18) zur Aufnahme einer Ultraschall-Ortungseinrichtung (50) angebracht ist.

3. Medizinischer Arbeitsplatz nach den Ansprüchen 1 und 2, dadurch gekennzeichnet, dass eine Stoswellenerzeugungsvorrichtung (46) mit Koppelkissen (48) vorgesehen ist, die so im Bereich der im räumlichen Zentrum stehenden Vorrichtung (2, 6) angeordnet ist, dass ein Patient (34) mit dem mit Stosswellen zu behandelnden Körperteil auf der Patientenliege (34) direkt über das Koppelkissen (48) verfahren und daran luftblasenfrei angekoppelt werden kann.

4. Medizinischer Arbeitsplatz nach den Ansprüchen 1 bis 3, dadurch gekennzeichnet, dass die Patientenliege (34) aus mindestens zwei Teilen (36, 42) besteht, von denen mindestens ein Teil (36) am Tragarm (8) mit Bodenplatte (10) derartig befestigt ist, dass die Patientenliege (34) um eine Liegenquerachse (38) kippbar ist, so dass Kopf-(36) und Fußteil (42) eine solche Aussparung aufweist, dass durch diese Aussparung hindurch ein Koppelkissen (48) unmittelbar an den Körper eines Patienten (30) luftblasenfrei ankoppelbar ist.

5. Medizinischer Arbeitsplatz nach einem der vorherigen Ansprüche, dadurch gekennzeichnet, dass der Tragarm (18) zur Aufnahme einer Ultraschallortungseinrichtung (50) längenveränderlich mit mindestens drei weiteren Armgliedern (20 bis 26) und einem am letzten Glied befestigten Ultraschallkopf (28) vorgesehen ist, dessen Bewegungsfreiheit derartig ausgestaltet ist, dass sämtlich zur Behandlung anstehenden Körperteile eines auf der Patientenliege (34) liegenden Patienten (30) mit dem Ultraschallkopf (28) erreichbar sind.

6. Medizinischer Arbeitsplatz nach einem der vorherigen Ansprüche, dadurch gekennzeichnet, dass eine Röntgenortungs-/Röntgendiagnostikanlage (56) vorgesehen ist, die soweit von der im räumlichen Zentrum stehenden Vorrichtung (2, 6) entfernt steht, dass mit dem an dem über und unter den Patienten (30) reichenden, bogenförmigen Arm befestigten Röntgenaufnahmegerät (72) sämtliche zur Behandlung anstehenden Körperteile erreichbar und aus frei wählbaren Richtungen zu durchleuchten sind.

7. Medizinischer Arbeitsplatz nach Anspruch 6, dadurch gekennzeichnet, dass die Halterung (66) der Röntgenanlage (56) um die senkrechte z-Achse (62) des Ständers (60) herum drehbar und um die senkrecht dazu liegende u-Achse (70) kippbar gelagert ist und das am Ende des bogenförmigen Armes (68) befestigte Röntgenaufnahmegerät (72) mit dem Armbogen (68) in der Halterung (66) um die Patientenlängsachse (16) herum schwenkbar ist.

8. Medizinischer Arbeitsplatz nach einem der vorherigen Ansprüche, dadurch gekennzeichnet, dass wenigstens ein Teil (42) der Patientenliege (34) von dem oder den anderen Teile (36) der Liege (34) und/oder dem Tragarm (8) lösbar und durch Beinstützen (76, 78) ersetzbar ist.

9. Medizinischer Arbeitsplatz nach einem der vorherigen Ansprüche, dadurch gekennzeichnet, dass Vorrichtungen zur Aufnahme der entstandenen Röntgen-oder Ultraschallbilder vorgesehen sind.

Fig. 1

Fig. 2

Fig. 3

Fig. 4

0 288 698

Fig. 5

Fig. 6